Europäisches Patentamt

European Patent Office

Office européen des brevets

(19)

(11) Publication number: **0 170 440**
**B1**

(12) # EUROPEAN PATENT SPECIFICATION

(45) Date of publication of patent specification: **19.09.90**

(21) Application number: **85304871.8**

(22) Date of filing: **08.07.85**

(51) Int. Cl.⁵: **H 01 B 7/34,** H 01 B 7/28,
H 01 B 3/10

(54) **Temperature resistant coated wire.**

(30) Priority: **08.07.84 IL 72333**
**09.07.84 GB 8417504**
**14.01.85 GB 8500816**

(43) Date of publication of application:
**05.02.86 Bulletin 86/06**

(45) Publication of the grant of the patent:
**19.09.90 Bulletin 90/38**

(84) Designated Contracting States:
**AT BE CH DE FR GB IT LI NL SE**

(56) References cited:
**EP-A-0 132 343**
**CH-A- 587 548**
**GB-A-1 133 333**

(73) Proprietor: **RAYCHEM LIMITED**
**Rolls House 7, Rolls Buildings Fetter Lane**
**London, EC4 1NL (GB)**

(72) Inventor: **O'Brien, James Martin**
**1 Osprey Close**
**Swindon Wiltshire (GB)**
Inventor: **Penneck, Richard John**
**"Treeve" Westway**
**Lechlade Gloucestershire (GB)**
Inventor: **Duckworth, Stephen John**
**5 Austen Crescent Liden**
**Swindon Wiltshire (GB)**
Inventor: **Smith, Nicholas John Gregg**
**50 Austen Crescent Liden**
**Swindon Wiltshire (GB)**

(74) Representative: **Dlugosz, Anthony Charles et al**
**Raychem Limited Intellectual Property Law**
**Department Faraday Road Dorcan**
**Swindon, Wiltshire (GB)**

Courier Press, Leamington Spa, England.

## EP 0 170 440 B1

**Description**

This invention relates to electrical wires that are formed from copper or copper alloys, and especially to such wires that may be subjected to high temperatures.

For example so called "magnet wire" which is used in electromagnet windings in transformers, motors and other equipment, may experience severe temperature excursions under overload conditions in service. Also, in certain fields where cables are used, for example in military or marine applications, it is desired to use cables that are capable of functioning for a period of time during a fire without failing. Such cables are called circuit integrity cables or signal integrity cables depending on their use. The previously proposed circuit and signal integrity cables have generally used the principle that the individual conductors should be separated from one another by mica tapes or by large volumes of packing materials or silicones or by combinations thereof in order to prevent the formation of short circuits during a fire, with the result that the previously proposed cables are relatively heavy or large or both.

It has been proposed to provide wires and cables that are intended to function at high temperatures with a thin insulating refractory coating, for example of alumina. For example, CH—A—587,548 describes a method of depositing alumina by chemical vapour deposition onto a copper surface that has been provided with a thin metallic keying layer formed for example from aluminium, manganese or zirconium. In addition, GB 8,318,612 (which is published on the file of EP—A—312,343 and which forms prior art under Art 54(3)) discloses an electrical wire having a refractory layer and polymeric insulation, in which the refractory layer is supported on a thin metallic intermediate layer. However we have found that, although the electrical properties of such refractory coatings may be satisfactory, when the articles are exposed to high temperatures the stability and integrity of the coating can be adversely affected such that the ability of the article to operate for extended periods is severely reduced.

According to the present invention there is provided an electrical wire which has a conductor formed from metallic copper, the conductor having on its surface an adherent substantially contaminant-free refractory layer and an intermediate layer formed from a metal which acts as a barrier to diffusion of oxygen or copper or both, the intermediate layer having a thickness of at least 1.5, especially at least 2 and most especially at least 3 micrometres, the wire also having an outer polymeric insulation.

It has been found that, in wires according to the invention the metal forming the intermediate layer eliminates or substantially reduces the mechanisms by which failure occurs, thus extending the high temperature lifetime of the article. Thus, for example in the case of circuit or signal integrity cables the time required to cause circuit failure in a fire would be substantially increased. The metal forming the intermediate layer for this purpose may be one which acts as a barrier to diffusion of either the underlying substrate to the outer surface of the article or to the diffusion of oxygen into the substrate. It may restrict diffusion in its elemental form or it may hinder diffusion processes, by formation of oxide scales when exposed to air, as is the case with for example aluminium. Such scales are most effective if they are stable on formation and exhibit low growth rates. The intermediate layer may be formed of metals which will alloy with the underlying substrate on exposure to high temperatures but which would still preferentially oxidise to form stable scales on exposure to air, or may be formed from metallic alloys which exhibit high oxidative stability e.g. titanium/aluminium alloys. The metal forming the intermediate layer may also be selected to take advantage of physical or chemical compatibility with the substrate and refractory layers to maximise adhesion.

In addition it has been found that in many cases the provision of a relatively thick intermediate layer significantly reduces the formation of cracks in the refractory layer when the wire is subjected to mechanical abuse. It is believed that the reduction in formation of cracks is due to the change in stress distribution in the refractory layer when the article is subjected to strain by virtue of the deformation of the intermediate layer. Thus, according to another aspect, the present invention provides an electrical wire which has a conductor formed from metallic copper, the conductor having on its surface an adherent substantially contaminant-free refractory layer and an adherent intermediate layer formed from a metal that has a lower modulus than that of copper (for example aluminium), the intermediate layer having a thickness of at least 1.5 micrometres, the wire also having an outer polymeric insulation. Since the absolute value of the modulus will depend on the strain, for strains beyond the limit of proportionality, and on the morphology of the material, the modulus as used herein refers to an arbitrary value of 1% strain and for the annealed material in its bulk form.

In some cases it is possible for a single metal intermediate layer both to have a lower modulus than that of copper and to act as a diffusion barrier to copper and/or oxygen, while in other instances more than one intermediate layer may be provided, for example one layer acting as a diffusion barrier and the other layer acting as a stress relieving layer or keying layer. For example a copper article may be provided with a nickel diffusion barrier intermediate layer and, on the nickel layer, an aluminium stress relieving/keying layer.

As stated above, the refractory layer is substantially contaminant-free, that is to say, the refractory layer contains only those species that are intended in order for the layer to fulfill its intended function, and contains substantially no species that result from the manufacturing process. An important feature of the refractory layer is good control of composition to optimise the high temperature performance of the article. The composition is totally inorganic and therefore does not rely on conversion processes to occur during

2

EP 0 170 440 B1

exposure to normal or emergency high temperature service, as is the case for example in many mica filled or glass filled silicone resin systems. The composition is also improved by removing the use of polymeric binders to support inorganic materials which may be consolidated by firing processes to form the inorganic insulation. Similarly, articles in which the refractory coatings have been formed by electrochemical conversion of metal layers e.g. by anodising an aluminium layer, do not form part of the invention, such layers often being heavily contaminated with ionic residue from the electrolytic solutions e.g. sulphates from sulphuric acid anodisation processes. Such wet chemical processes may result in contamination of the intermediate layer described above. Suitable deposition techniques include physical vapour deposition processes such as reactive evaporation and sputtering or plasma assisted chemical vapour deposition. Coatings can also be formed by plasma oxidation of the metals or by non vacuum processes such as a high pressure CVD method, the refractory layer preferably being deposited at a temperature of not more than 350°C.

It has been found that wires according to the invention are highly resistant to high temperatures and that the integrity of the refractory coating is not destroyed at high temperatures for relatively long periods of time. By examination of articles in accordance with the present invention and articles in which no, or only a thin, intermediate metal layer is present, by means of a scanning electron microscope, it has been observed that the predominant failure mechanism of articles having no intermediate layer is through spalling. When articles are provided with a thicker metal intermediate layer the spalling is reduced and failure occurs through a mechanism in which the underlying copper appears to migrate through the refractory layer and appear at the outer surface of the refractory layer, in the form of small globules or a network of "dykes" or in other cases, in the form of "blisters". This form of failure may occur at temperatures as low as 500°C, well below the melting point of copper. The particular reason why this failure occurs is unclear and it is likely that more than one mechanism is responsible for the failure in different cases. One theory as to the failure mechanism is that, at elevated temperatures, the underlying copper is oxidized by ambient oxygen which has penetrated the refractory layer, either by diffusion or through cracks that may have been caused by mechanical or thermal stresses in the refractory layer, to form copper oxide ($Cu_2O$ or $CuO$) which are relatively electrically conductive. Growth of the copper oxide scale would proceed by outward diffusion of copper through the copper oxide to combine with inwardly diffusing oxygen until it reached the outer surface of the refractory layer. In the case of circuit integrity wires electrical integrity of the system would be significantly deleteriously affected.

Whatever the precise failure mechanism is, and whether the underlying copper migrates through the refractory layer in its elemental form or in the form of its oxide, it has been observed that this migration may be significantly reduced or prevented by the provision of a relatively thick intermediate layer which acts as a barrier to diffusion of oxygen or copper or both.

It has also been observed that thick intermediate layers can act to reduce or eliminate crack formation resulting from the thermal expansion mismatch between copper and the refractory layer, and so improve the temperature resistance of the article.

The conductor may be a single, solid conductor or it may be a stranded conductor in which individual strands are laid together to form a bundle which preferably contains 7, 19 or 37 strands. Where the conductor is stranded it is the bundle that is coated with the intermediate and refractory layers rather than the individual strands, that is to say, the intermediate and refractory coatings extend around the circumference of the bundle but not around the individual strands so that substantially only the outwarldy lying surfaces of the outermost layer of strands are coated. It is always possible for the stranded conductor to be formed from more than one metal e.g. in the case of conventional tin or nickel coated stranded conductors, but in this case any tin or nickel coating is present in addition to the intermediate layer.

This form of conductor has the advantage that the inner strand electrical contact is retained and the dimensions of the bundle are kept to a minimum (since the thickness of the coating may constitute a significant proportion of the strand dimensions for fine gauge conductors) and also it aids the formation of good electrical connections, e.g. crimp connections, to the conductor because a large proportion of the surface of the strands, and the entire surface of the strands in the central region of the conductor, will be uncoated by the refractory layer.

If a circuit or signal integrity cable is formed according to the invention from a stranded conductor, it has the advantage that it is very flexible as compared with other signal and circuit integrity cables, especially if a stranded conductor is used. The ability of the wire to be bent around very tight bends (small bend radii) without deleterious effect is partly due to the fact that the layer providing the integrity is thinner than with other signal and circuit interity cables. However, when the conductor is a stranded conductor it may be bent around extremely tight bends without undue stress on the surface of the strands because the strands are displaced from a regular hexagonal packing at the apex of the bend thereby exposing uncoated areas of the strands to the eye. It is highly surprising that even though uncoated strands may be exposed when the wire conductor is bent there is no electrical contact between adjacent stranded conductors. It is believed that in this case the integrity is retained because the profile of a stranded conductor is not cylindrical but rather is in the form of a hexagon that rotates along the length of the conductors, so that adjacent stranded conductors will touch one another only at a few points along their length, which points are always provided by the outwardly oriented part of the surface of the strands in the outer layer of the conductors. It is these points of contact that are always provided with the refractory coating.

The refractory coating preferably has a thickness of at least 0.5, more preferably at least 1 and especially at least 2 micrometres but preferably not more than 15 and especially not more than 10 micrometres, the most preferred thickness being about 5 micrometres depending upon specific operational requirements. The exact thickness desired will depend on a number of factors including the type of layer and the voltage rating of the wire, circuit integrity cables usually requiring a somewhat thicker coating than signal integrity cables and sometimes above 15 micrometres. The lower limits for the coating thickness are usually determined by the required voltage rating of the wire whilst the upper limits are usually determined by the time, and therefore the cost, of the coating operation.

Preferably the insulating refractory coating is formed from an electrically insulating infusible or refractory metal or semi-metal oxide or nitride and the invention will be described below in many cases with respect to oxides and nitrides although other refractory coatings are included. By the term "infusible" or "refractory" is meant that the coating material in its bulk form should not fuse or decompose when subjected to a temperature of 800°C, for 3 hours. Preferably the oxide or nitride should be able to withstand higher temperatures also, for example it should be able to withstand a temperature of 1000°C for at least 20 to 30 minutes. The preferred oxides are those of aluminium, titanium, tantalum and silicon or mixtures thereof with themselves or with other oxides, and the preferred nitrides are those of aluminium and silicon. Thus, for example, the use of mixed metal oxides for the refractory coating are also encompassed by the present invention. It should be appreciated that the oxide or nitride layer need not, and in many cases will not, have a precisely defined stoichiometry. In a number of cases, depending on the method of forming the refractory coating, the coating will contain the metal or semi-metal in a stoichiometric excess, that is to say, the coating will contain more metal than is required for the stoichiometry of a defined formal oxidation state of the metal. Accordingly the terms "aluminium oxide", "titanium oxide", "tantalum oxide", "silicon oxide", "metal oxide" and the equivalent terms when referring to nitrides are intended to include non-stoichiometric compounds. It is often advantageous for the refractory coating to be non-stoichiometric since this may increase the adhesion between the refractory coating and the underlying layer, and especially if the stoichiometry of the refractory coating varies through at least part of its thickness so that stresses that may be induced in the coating, for example due to differential thermal expansion, are not localised to a boundary of the coating and so that different parts of the coating will exhibit different properties. For example, a relatively metal-rich part of the coating may exhibit good adhesion to the conductor or intermediate layer while part of the coating having least metal or semi-metal may exhibit the best electrical properties.

If desired, the stoichiometry of the refractory coating may vary continuously throughout the thickness of the coating or it may contain one or more layers or strata of relatively uniform stoichiometry. Thus the coating may have an outer region of relatively uniform stoichiometry and preferably of a relatively high oxygen or nitrogen content in order to exhibit the optimum electrical properties. The relative thicknesses of the non-uniform and uniform layers may vary widely. For example the major part of the coating may have a non-uniform stoichiometry or the major part of the coating's thickness may be of uniform stoichiometry, in which latter case the non-uniform part of the coating could even be considered as an intermediate layer that improves adhesion of the coating especially at high temperatures. If the underlying metal or semi-metal-rich part of the coating is intended to improve the adhesion of the refractory coating, its particular composition will depend on the composition of any underlying layer, and in some cases it may be desirable for the metal or semi-metal rich part to consist substantially entirely of the metal or semi-metal so that there is a gradual change from the metal or semi-metal to the oxide or nitride. This is particularly preferred if the system includes an intermediate layer of the same metal or semi-metal.

The precise stoichiometry of the uniform top layer can be determined experimentally using wavelength dispersive electron microprobe analysis or by using X-ray photoelectron spectroscopy (XPS). The composition of the coating as it changes from metal to refractory throughout its depth can be assessed using Auger electron spectroscopy (AES) in which the film is continuously sputtered away to expose fresh surfaces for composition analysis.

The variation in stoichiometry is not limited to a variation in the metal or semi-metal/oxygen or nitrogen proportions. In addition or alternatively the relative proportions of two different metals or semi-metals may be varied so that, for example, there is a gradual change from one metal, which may constitute an intermediate layer, to the oxide or nitride of a different metal.

The outer region of the refractory coating preferably has a molar oxygen or nitrogen content that is at least 50%, more preferably at least 65% and especially at least 80% of the oxygen or nitrogen content of a defined stable formal oxidation state of the metal. Thus the preferred oxide composition of the outer region may be represented as $MO_x$

where x is at least 0.75, preferably at least 1 and especially at least 1.25 in the case of aluminium,
at least 1, preferably at least 1.3 and especially at least 1.5 in the case of titanium or silicon, and
at least 1.25, preferably at least 1.6 and at least 2 in the case of tantalum.

For relatively thin refractory coatings that have a stoichiometric excess of the metal or semi-metal it has been found that the coating remains insulating as the temperature is raised up to a certain temperature, usually in the range of 300 to 600°C and then becomes conductive when a load of 30 V is applied. In general the electrical properties of the coatings, as determined by the temperature of onset of conductivity, may be improved both by increasing the thickness of the coating and by increasing the oxygen or nitrogen content thereof although to some extent either the thickness or the oxygen or nitrogen content may be increased at the expense of the other.

Although it is possible, at least in the broadest aspect of the invention, for the refractory coating to consist of a single layer only which is deposited on the intermediate layer it is possible, and in many cases preferable, for one or more additional layers to be formed. For example a refractory coating comprising an oxide may have a refractory nitride layer thereon. Examples of nitrides that may be deposited on refractory coatings to improve the mechanical properties include titanium nitride or aluminium nitride.

Other examples of additional layers that may be employed are refractory intermediate layers or further metallic intermediate layers located between the conductor and the refractory oxide or nitride coating as mentioned above. In order to improve the adhesion between the refractory coating and the conductor it is preferred for the metallic intermediate layer, adjacent to the refractory coating to be formed from that metal from which the refractory coating is formed. Preferred metallic intermediate layers include those formed from nickel, aluminium, titanium, tantalum or silicon although other metals, e.g. chromium, manganese or silver may be used.

More than one intermediate layer may be provided on the conductor if desired, for instance a barrier layer may be provided between the conductor and the other intermediate layer or an alloy layer may be formed from a deposited metal and the conductor metal (e.g. Aluminium/copper) during manufacture, or in a subsequent heating step or in high temperature use. In at least some cases the provision of an alloy layer significantly improves adhesion of the coating.

In the case of electrical equipment the refractory layer may provide the entire electrical insulation or one or more additional insulating layers may be provided thereon. The additional insulating layer may be inorganic or organic or a combination of inorganic and organic layers may be provided.

All the compositions, structures and processes described herein are applicable to such articles.

The polymeric insulation is provided in order to provide additional insulation to the conductor during normal service conditions and also to enable the wire to have the desired dielectric properties and other properties e.g. mechanical properties, scuff resistance, colour coding ability etc. However, an important advantage of the present invention is that since a significant proportion of or all the service insulating properties are provided by the refractory coating, the electrical properties of the polymeric insulation are not as critical as with other wire constructions in which the polymeric insulation provides the sole insulation between the conductors. Of the known polymeric materials that are used for electrical insulation, polyethylene probably has the most suitable electrical properties but is highly flammable, and has poor mechanical properties. Attempts to flame retard polyethylene have either required halogenated flame retardants which, by their nature, liberate corrosive and toxic hydrogen halides when subjected to fire, or have required relatively large quantities of halogen free flame retardants which have a deleterious effect on the electrical properties and often also the mechanical properties of the polymer. Accordingly, an acceptable wire has in the past only been achieved by a compromise between different properties which is often resolved by using a relatively thick-walled polymeric insulation and/or dual wall constructions. Although such forms of polymeric insulation may be used with the wire according to the present invention, the presence of the refractory layer does obviate these problems to a large extent since the polymer used for the insulation may be chosen or its flammability and/or its mechanical properties at the expense of its electrical properties. As examples of polymers that may be used to form the polymeric insulation there may be mentioned polyolefins e.g. ethylene homopolymers and copolymers with alpha olefins, halogenated polymers e.g. tetrafluoroethylene, vinylidene fluoride, hexafluoropropylene and vinyl chloride homo or copolymers polyamides, polyesters, polyimides, polyether ketones e.g. polyarylether ketones, aromatic polyether imides and sulphones, silicones, alkene/vinyl acetate copolymers and the like. The polymers may be used alone or as blends with one another and may contain fillers e.g. silica and metal oxides for instance treated and untreated metal oxide flame retardants such as hydrated alumina and titania. The polymers may be used in single wall constructions or in multiple wall constructions, for example a polyvinylidene fluoride layer may be located on for example a polyethylene layer. The polymers may be uncrosslinked but preferably are crosslinked, for example by chemical cross-linking agents or by electron or gamma irradiation, in order to improve their mechanical properties and to reduce flowing when heated. They may also contain other materials e.g. antioxidants, stabilizers, crosslinking promotors, processing aids and the like. It is particularly preferred for the polymeric insulation to contain a filler e.g. hydrated alumina, hydrated titania, dawsonite, silica and the like, and especially a filler that has the same chemical composition, at least under pyrolysis conditions, as the refractory coating, so that the filler in the polymeric insulation will provide additional insulation when the wire or cable is subjected to a fire. Another preferred type of polymeric insulation is one that will char, for instance certain aromatic polymers mentioned above, or that will ash e.g. a silicone polymer, when subjected to a fire so that the char or ash, together with the refractory coating, will provide the necessary insulation during a fire. Examples of polymers, compositions, their manufacture and wires using them are described in U.S. Patent Specifications Nos. 3,269,862, 3,580,829, 3,953,400, 3,956,240, 4,155,823, 4,121,001 and 4,320,224, British Patent Specifications Nos. 1,473,972, 1,603,205, 2,068,347 and 2,035,333, 1,604,405 and in European Patent Specification No. 69,598, the disclosures of which are incorporated herein by reference. Preferably the wire is substantially halogen free.

The metallic intermediate layer may be formed in a number of ways, for instance by electroplating, standard wire cladding techniques such as roll bonding or by coating from a metal melt, and by vacuum deposition techniques e.g. sputtering, evaporation, flame spraying, plasma assisted chemical vapour deposition (CVD) or other techniques. Preferably the intermediate layer is formed at a temperature of not more than 150°C.

As stated above, the refractory layer may be formed by any of a number of methods provided that the particular method used will deposit a substantially contaminant-free layer. The layer may, if desired, be deposited by a non vacuum process, for instance a high pressure chemical vapour deposition method.

However a vacuum deposition method such as evaporation, plasma assisted chemical vapour deposition, or especially a sputtering method is preferred.

In the sputtering method, predominantly neutral atomic or molecular species are ejected from a target, which may be formed from the material to be deposited, under the bombardment of inert gas positive ions e.g. argon ions. The high energy species ejected will travel considerable distances to be deposited on the wire conductor substrate held in a medium vacuum, e.g. $10^{-4}$ to $10^{-2}$ mbar. The positive ions required for bombardment may be generated in a glow discharge where the sputtering target serves as the cathode electrode to the glow discharge system. The negative potential (with respect to ground and the glow discharge) is maintained in the case of insulating target materials by the use of radio frequency power applied to the cathode, which maintains the target surface at a negative potential throughout the process. DC power may be applied when the target is an electrically conducting material. The advantage of such techniques is that control of the target material is greatly enhanced, and the energy of the species ejected is very much higher than with evaporation methods e.g. typically 1 to 10 eV for sputtering as compared with 0.1 to 0.5 eV for evaporation methods. Considerable improvements in interfacial bonding are achieved but the deposition rate in the sputtering process described will be lower than that for electron beam evaporation.

In magnetron sputtering processes the plasma is concentrated immediately in front of the cathode (target) by means of a magnetic field. The effect of the magnetic field on the gas discharge is dramatic. In that area of discharge where permanent magnets, usually installed behind the cathode, create a sufficiently strong magnetic field vertically to the electric field, secondary electrons resulting from the sputter bombardment process will be deflected by means of the Lorenz force into circular or helical paths. Thus the density of electrons immediately in front of the cathode as well as the number of ionised argon atoms bombarding the cathode are substantially increased. There is an increase in plasma density and a considerable increase in deposition rate. Bias sputtering (or sputter ion plating) may be employed as a variation of this technique. In this case the wire conductor is held at a negative potential relative to the chamber and plasma. The bombardment of the wire conductor by Argon ions results in highly cleaned surfaces. Sputtering of the target material onto the wire conductor throughout this process results in a simultaneous deposition/cleaning mechanism. This has the advantage that the interfacial bonding is considerably improved. In sputter ion plating systems both substrate and the wire conductor are held at a negative potential. In this case the relative potentials are balanced to promote preferential sputtering of the target material. The target voltage will be typically less than 1 kV, dependent on system design and target material. The wire substrate, may be immersed in its own localised plasma dependent upon its bias potential, which will be lower than that of the target. The exact voltage/power relationship achieved at either target or substrate is dependant upon many variables and will differ in detail from system to system. Typical power densities on the target are 10—20 W/cm$^2$. The load to the substrate may be substantially lower, often as little as 5% of the target load.

The preferred technique that is used to apply the oxide or nitride coating is a reactive bias sputtering method in which the reactive gas is introduced into the vacuum chamber in addition to argon so that the oxide/nitride of the target material, which in this case is a metal or semi-metal rather than the oxide/nitride will be deposited. Experimental results have shown that the level of reactive gas and its admission rate have a significant effect on deposition rates. The precision control of partial pressure of the reactive gas and the analysis of the sputtering atmosphere in a closed loop control system is considered highly desirable. Apart from the simultaneous deposition/cleaning advantages mentioned above, the ion bombardment of the substrate enhances surface reaction between the reactive gas and depositing species, resulting in more efficient formation of the coating with the required stoichiometry.

Partial pressure of reactive gas is determined experimentally but will normally be between 2 and 25% but sometimes up to 30%, the exact level depending on the required stoichiometry of the coating and deposition rate. Reactive sputtering is also the preferred technique because it facilitates alterations to the stoichiometry of the coating. For example, an intermediate "layer" of the pure metal used for the oxide/nitride coating may be deposited in such a way that there is no defined boundary between the conductor metal, oxide/nitride metal and oxide/nitride layers.

The vacuum chambers and ancillary equipment, including micro-processor gas control units and a variety of targets used in these methods may be purchased commercially. Many variations in design are possible but most employ the use of "box" shaped chambers which can be pumped down to high vacuum for use in any of the vacuum deposition processes mentioned. Systems are normally, but not exclusively, dedicated to one deposition process. One system which may be employed to coat wire uses air to air transfer techniques for passage of the wire conductor through the deposition chambers and employs one or more ancillary vacuum chambers either side of the main deposition chamber.

These ancillary chambers are held at progressively higher pressures as they extend from deposition chamber to air. This reduces the load on individual vacuum seals. The system described has the advantage of continuous feed of the wire conductor over batch process arrangements. In the vacuum deposition chamber the pressure is held constant at a pressure normally between $10^{-4}$ and $10^{-2}$ Torr.

The targets employed are commercially available Planar Magnetron Sputtering sources. Their size may vary widely, and targets in excess of 2 metres in length may be employed. Between two and four such sources may be arranged opposite one another so as to surround the wire conductor passing through the chamber or to sputter from at least two sides. The arrangement may be employed in series to increase wire throughout rates. As described above a negative bias is applied to the magnetron to initiate the sputtering process. The wire may be held at a lower negative bias as described earlier.

Refinements to the system can, if desired, be employed. For example, the use of an intermediate vacuum station between the air (input side) and the deposition chamber may be employed to generate an Argon ion glow discharge which cleans the wire conductor surface by ion bombardment prior to its entry into the vacuum deposition chamber and also heats the wire conductor.

Further intermediate chambers can be employed between the cleaning and deposition chamber to deposit intermediate layers.

Conditions may be controlled to produce any of the conductor coatings described above in which no defined boundaries occur between the layers. For example an intermediate "layer" of the pure metal used for the refractory coating may be deposited in such a way that there is no defined boundary between the conductor metal, the intermediate layer and the oxide or nitride coating. In a similar fashion additional chambers can be employed between the deposition chamber and air (output side) to deposit different metal, metal oxide or metal alloys onto the refractory coating for improved lubrication or wear resistance.

Evaporation and the related processes of activated evaporation and ion plating offer alternative technical for deposition of the coating, with significant advantages in deposition rate.

Evaporation of the coating material is achieved by heating the material such that its vapour pressure exceeds $10^{-2}$ mbar. Evaporation temperatures vary according to coating material, e.g. 1300—1800°C for refractory metal oxides, the chamber pressure being usually $10^{-4}$ to $10^{-6}$ mbar. Similar wire transport systems to those described may be used to hold the substrate about 30—40 cm above the source. Several heating methods exist e.g. resistive, inductive, electron beam impingement etc. although the preferred method is an electron beam source where a beam of high energy electrons e.g. 10,000 eV impinge onto the coating material contained in a water-cooled crucible. The use of multi-pot crucibles or twin source guns, enable multiple layers and graded stoichiometry layers to be deposited with the aid of electronic monitoring and control equipment.

Compound coatings can be made either by direct evaporation from that compound e.g. $Al_2O_3$ or by reactive evaporation, e.g. aluminium evaporated into a partial pressure of oxygen to give aluminium oxide. Variations in the process exist either to promote reactions or adhesion, e.g. Activated reactive evaporation (ARE) can be used to increase the reaction probably between the evaporant and the reactive gas.

In ion-plating, negative bias applied to the substrate in an inert gas, promotes simultaneous cleaning/deposition mechanisms for optimising adhesion as described in the sputtering process. Bias levels of $-2$ KV are typically used but these can be reduced to suit wire substrates. Alternatively, high bias can be applied to a plate positioned behind the traverse wire to achieve a similar effect. As operating pressures are higher in the ion plating technique, e.g. $10^{-3}$ to $10^{-2}$ mbar, gas scattering results in a more even coating distribution. To protect the filament the electron beam gun in the ion plating technique is differentially pumped to maintain vacuum higher than $10^{-4}$ mbar.

In the Plasma assisted chemical vapour deposition (PACVD) method the substrate to be coated is immersed in a low pressure (0.1 to 10 Torr) plasma of the appropriate gases/volatile compounds. This pressure is maintained by balancing the total gas flow-rate against the throughput of the pumping system. The plasma is electrically activated and sustained by coupling the energy from a power generator through a matching network into the gas medium. Thin films have been successfully deposited from direct current and higher frequency plasmas well into the microwave range. At high frequencies the energy may be capacitatively or inductively coupled depending on chamber design and electrode configuration. Typically a 13.56 MHz radio-frequency generator would be used having a rating which would allow a power density of between 0.1—10 W/cm² in a capacitatively coupled parallel-plate type reactor. The substrate, which could be set at a temperature of up to 400°C, may be grounded, floating or subjected to a dc voltage bias as required. Typically deposition rates for this technique can be favourably compared with those obtained by sputtering. The deposition of alumina may be achieved by immersing a substrate in a plasma containing a volatile alumina compound (e.g. Tri-methyl aluminium or Aluminium butoxide) and oxygen under appropriate processing conditions.

After the oxide coating has been deposited on the wire conductor the polymeric insulation may be extruded into the coated conductor by methods well known in the art.

In order to form a circuit or signal integrity on cable the appropriate wires according to the invention may simply be laid together and be enclosed in a jacket. If desired the wires may be provided with a screen or electromagnetic interference shield before the cable jacket is applied. Thus a cable may be formed in a continuous process by means well known in the art by braiding the wire bundle and extruding a cable jacket thereon. Any of the materials described above for the wire polymeric insulation may be used

7

although halogen-free compositions e.g. compositions as described in the U.K. Patent Specifications Nos. 1,603,205 and 2,068,347A mentioned above are preferred. It is of course possible to employ additional means for providing integrity of the cable such as mica tape wraps, but these are not necessary nor are they desirable in view of the increased size and weight of the cable.

In certain circumstances it may be desirable to coat the oxide layer with a thin coating of a polymeric resin or lacquer in order to provide a barrier against water or electrolytes during service.

The present invention is especially suitable for forming flat cables which, as will be appreciated, are not susceptible to being wrapped with mica tape.

Several embodiments of the invention and a method of production thereof will now be described by way of example with reference to the accompanying drawings in which:

Figure 1 is a cross-section through one form of wire according to the present invention;

Figure 2 is a cross-section through a signal integrity cable employing the wires of Figure 1;

Figure 3 is a cross-section through part of a flat conductor flat cable;

Figure 4 is a schematic view of part of the sputtering apparatus showing its wire handling mechanism;

Figures 5 and 6 are schematic sections through part of the thickness of article in accordance with the invention; and

Figure 7 is a graphical representation showing improved crack resistance of articles in accordance with the invention.

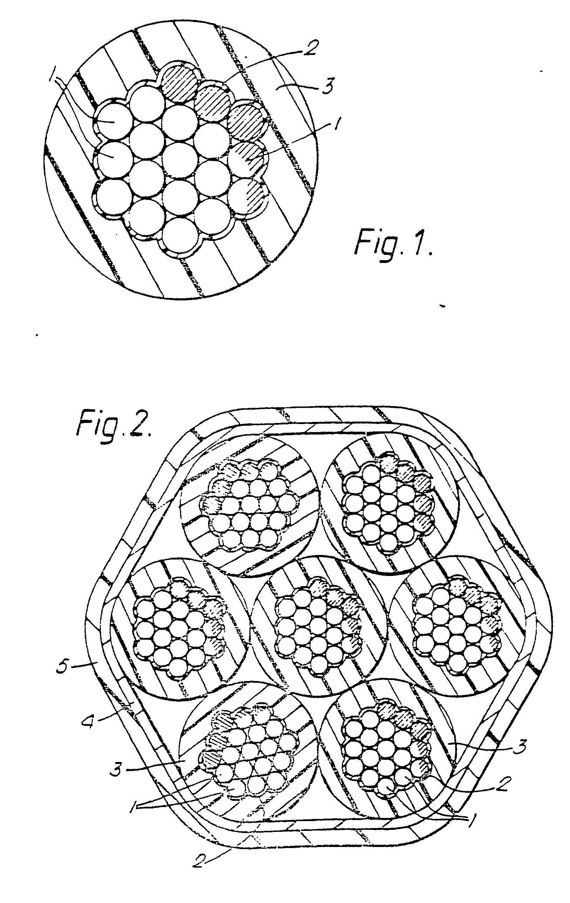

Referring to Figure 1 of the drawings a 26 AWG stranded copper conductor formed from 19 copper strands 1 is coated with a 5 micrometre thick layer 2 of aluminium oxide by the sputter ion plating method described above. Before deposition of the aluminium oxide, the outer surface of the stranded conductor was provided with a 3 micrometre thick layer of aluminium (not shown). A coating 3 based on a polyetherimide sold under the trade name "Ultem" or a polyether ether ketone or polyether ketone is then extruded on the oxide coated conductor to form a polymeric "insulating" layer of mean wall thickness 0.2 mm.

Figure 2 shows a signal integrity cable formed by laying together seven wires shown in Figure 1, forming an electromagnetic interference screen 4 about the bundle by braiding and then extruding thereon a jacket 5 based on a halogen-free composition as described in British Patent Specification No. 2,068,347 Example 1A.

The cable so formed is particularly lightweight and has a relatively small overall diameter in relation to the volume of the copper conductor.

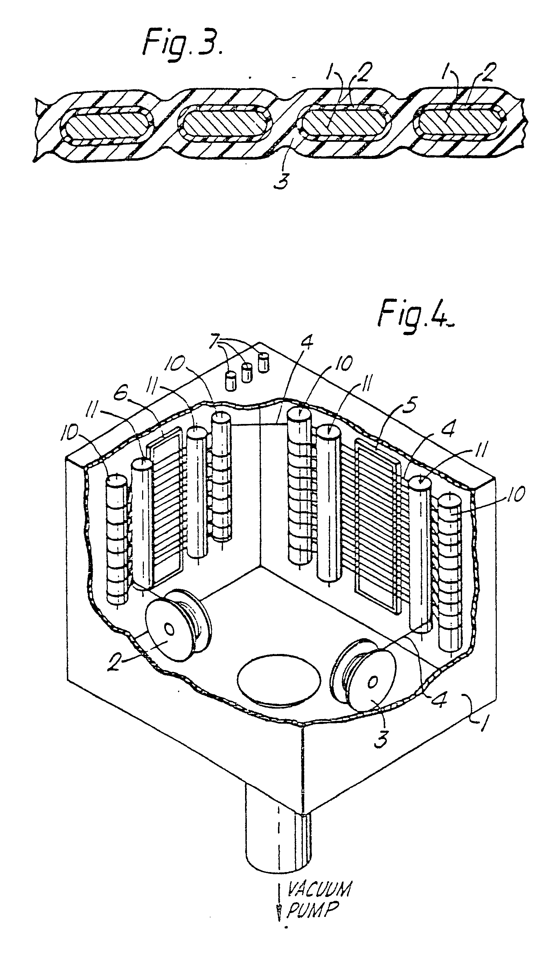

Figure 3 shows a flat conductor flat cable comprising an array of flat copper conductors 1 with a 100 mil (2.54 mm) spacing. Each copper conductor 1 is provided with a 3 micrometre thick aluminium intermediate layer and a 5 micrometre thick alumina layer thereon as described above, and the coated conductors are embedded in a single polymeric insulating layer formed for example from the polyether imide sold under the trade name "Ultem" or from a polyether ether ketone or polyether ketone.

Apparatus for use in a batch process for coating wire conductor substrate is illustrated in Fig. 4. The apparatus comprises a vacuum chamber into which a complete wire transport mechanism which includes wire pay-off reel 2 and take-up reel 3, wire support rolls 10 and tensioning rolls 11 is loaded. The mechanism engages motor drives which control the passage of wire 4 so that the wire traverses a vertically mounted target 5 a number of times. Deposition occurs by the processes previously described. As before, variations in set-up are possible. An additional target (not shown) may be employed on the other side of the wire to increase coating rates and additional targets, e.g. target 6 can be employed to deposit intermediate layers before and/or after deposition of the primary oxide/nitride coating. Suitable design of the gas inlet system to suit the specific geometries employed can facilitate deposition of layers which have no defined boundaries as described previously. Batch length will depend on chamber dimensions and transport system design.

In the operation of such a batch process wire 4 is transferred from one reel 2 to the other 3 within the chamber. The route taken by the wire may cause it to pass before the smaller ancillary target 6 to deposit an intermediate layer of any desired material. Power to this target, combined with wire speed and the number of passes in front of the target will control the thickness of the intermediate layer deposit. The wire 4 may then pass in front of the larger primary target 5 to deposit the main coating. Again thickness will be dictated by a combination of power, wire speed and a number of passes. The ratio of thicknesses between the intermediate and the primary coating is controlled in the same way. Multilayers can be built up by reversing passes back the targets 5, 6 in reverse order. Thickness and composition may be altered in the reverse pass as required, e.g. the process employed at the smaller magnetron may be reactive on the reverse pass to deposit a compound of the metal on the intermediate layer, e.g. Ti and $TiN_x$. Deposition of layers with no defined boundary between the metal intermediate layers (or substrates) and the oxide/nitride coatings may be achieved by setting up gradients of reactive gas in front of the primary target, such that wire at the top edge of the target 5 is subjected to deposition in an Argon rich atmosphere which gradually increases in reactive gas content as the wire progresses down the face of the target. A gradient can be achieved by a baffle system (not shown) which progressively leaks oxygen introduced at the bottom end of the target towards the upper end.

A simpler technique for producing the layer with no defined boundary involves use of a multipass process in which wire 4 is passed back and forth through the system, and with each pass the level of reactive gas is increased to a final level required to obtain the correct stoichiometry. Thus the stoichiometry of the intermediate layer increases in a series of small incremental steps from metal to required stoichiometry. Composite targets may also be used to produce intermediate layers with stoichiometry gradients. In the case of discrete articles, the articles may instead be held in front of the target by means of a rotating sample holder.

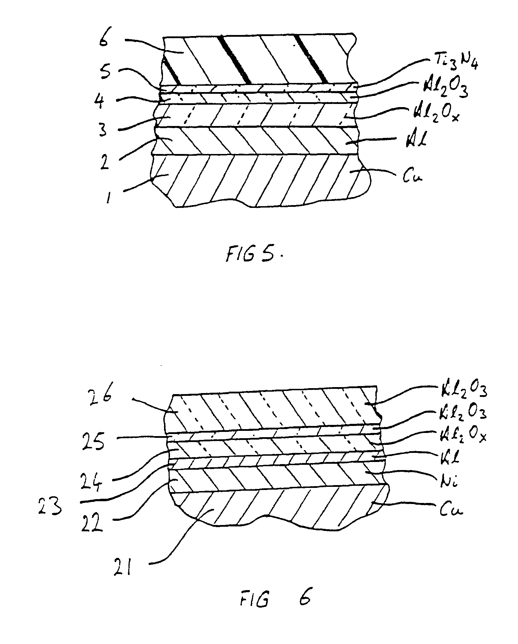

Figures 5 and 6 are schematic sections through parts of articles according to the invention showing typical arrangements of layers that may be formed on the copper substrate, the thickness of the layers being exaggerated for the sake of clarity.

As shown in Figure 5 a copper substrate 1 is provided with a layer 2 of aluminium metal of a thickness of about 2 to 10 micrometres which may have been deposited for example by sputtering, electrodeposition or from the melt. A layer 3 of aluminium oxide $Al_2O_x$ is provided on the aluminium layer, the value of x varying from 0 in the region adjacent to the aluminium layer to 3 at the top surface, and a thin layer 4, e.g. about 3 micrometres in thickness, of stoichiometric aluminium oxide is deposited on the top surface of the aluminium layer. A further, thin, layer 5 of titanium nitride may be provided in order to increase the toughness of the article, followed by a relatively thick organic polymer layer 6.

Although the layers are clearly demarcated in the drawing by means of lines, it will be appreciated that such boundaries may, and preferably will, not be formed in practice, especially between the copper/ aluminium, aluminium/$Al_2O_x$ and $Al_2O_x$/$Al_2O_3$ layers. Indeed, the aluminium, $Al_2O_x$ and stoichiometric alumina layers may all be formed in the same sputtering process in which case the stoichiometry of the layers will depend on the oxygen gradient used.

Another typical example is shown in Figure 6 in which a copper substrate 21 is provided with a thick (e.g. 1 to 3 micrometres) layer 22 of nickel followed by a layer 23 of aluminium metal, a layer 24 of non-stoichiometric aluminium oxide $Al_2O_x$ and a layer 25 of stoichiometric aluminium oxide $Al_2O_3$, the layers 23, 24 and 25 having been formed e.g. by a sputtering method. An additional, relatively thick layer 26 of aluminium oxide (e.g. of about 5 to 15 micrometres thickness) may be deposited on the layer 25 by a non-vacuum deposition method.

The following Examples illustrate the invention:

Examples 1 to 7

Copper conductors were provided with aluminium intermediate layers of various thicknesses by use of the sputtering apparatus shown schematically in Figure 4 of the drawings. The sputtering conditions were as follows: the wire 4 was precleaned by vapour degreasing in 1,1,1-trichloroethane prior to deposition. The cleaning was achieved by passing the wire through a vapour degreasing bath such that a residence time of 3 minutes was achieved. The wire 4 was then loaded into the vacuum chamber. The chamber was then evacuated to a pressure of $1 \times 10^{-6}$ mbar prior to starting the process. At this stage argon was admitted to attain a pressure of $1.5 \times 10^{-2}$ mbar whereupon a high frequency (80 kHz) bias potential was applied to the wire handling system which was isolated from ground. A bias potential of $-850$ V was achieved, and the wire was transferred from reel 3 to reel 4 such that a residence time of 10 minutes was achieved. On completion of the cleaning cycle the pressure was reduced to $8 \times 10^{-3}$ mbar and the deposition process started.

3 kW of DC power was applied to the aluminium target 5. The wire passed from reel 2 to reel 3 being coated as it passed the target 5. Residence time in this region was controlled by wire speed and adjusted to give the required thicknesses. The roller mechanism alternated the wire face exposed to the target as it progressed down the target length.

Samples of copper conductor coated with aluminium as described above were subsequently coated with aluminium oxide in a similar process. For this second coating, an aluminium oxide target powered with an RF power supply was used. The wire residence time and target power were adjusted to give a constant thickness of aluminium oxide, being about 4 micrometres. During deposition of both aluminium and aluminium oxide the copper conductors were held at a bias potential relative to teh chamber to promote adhesion. The D.C. dielectric strength of the refractory aluminium oxide layer was measured to be 57 volts/micrometre.

The electrical performance of the insulated wires so formed was tested by twisting a pair of identical wires (2 twists per 2.5 cms length) to form a twisted pair cable, connecting one end of the wires to a 1 MHz, 30 V square wave source and observing the wave across a 200 ohm load at the other end of the wires by means of an oscilloscope. The twisted pair cables were subjected to heating in a propane gas burner having a flat flame 8 cm wide. The temperature of the flame just below the twisted pairs was maintained at 900°C and the time to failure recorded.

The results are given in Table 1, from which it may be seen that wires provided with intermediate layers beneath the refractory coating according to the invention exhibit significantly greater times to failure:

## EP 0 170 440 B1

TABLE 1

| Example number | Aluminium intermediate layer thickness (micrometres) | Time to failure in an 900°C propane flame (minutes) |
|---|---|---|
| 1 | 0 | 0.2 |
| 2 | 1 | 2.5 |
| 3 | 2 | 3.5 |
| 4 | 2.5 | 9.0 |
| 5 | 3.3 | 32 |
| 6 | 6.2 | 94 |
| 7 | 11 | 360* |

\* Test determined after 360 minutes with no failure observed.

The twisted pair samples were examined using optical and scanning electron microscopes after completion of the propane flame test. In the case of thicker intermediate layers, failure seemed to have occurred by migration of copper to form copper oxide, as described in the text. However, it was apparent that as the intermediate layer thickness increased, the circumferential cracking of the aluminium oxide decreased to the point where it was not detectable under 200× magnification. This reduction in crack formation is surprising in view of the fact that the aluminium intermediate layer has a higher thermal expansion mismatch with the aluminium oxide than does copper. In these cases migration proceeded either by slow diffusion through the refractory layer or by diffusion between wire strands. This progressive change in failure mode is reflected in the survival time observed.

Example 8

7 Strand 20 AWG copper conductors were provided with a composite coating consisting of a first layer of 2 micrometres of titanium, a second layer of graded stoichiometry $TiO_x$ in which x varied from 0 to 2 over a thickness of approximately 2 micrometres and a third layer of 2 micrometres of $TiO_2$.

The composite coatings were deposited by the use of reactive sputtering techniques and apparatus as described above.

The coated conductors were then provided with a 0.25 mm insulating layer of low density polyethylene which was crosslinked by irradiation with high energy electrons to a dose of 20 Mrads.

Twisted pairs of these coated conductors were heated to 900°C for 10 seconds. It was observed that the coating remained intact, with no spalling and no copper migration through the layer, even though the polymer insulation had burned away.

Examples 9 to 12

19 Strand, 22 AWG copper wire samples were provided with aluminium intermediate layers of different thicknesses as described in Examples 1 to 7, followed by an aluminium oxide layer 4 micrometres thick.

The samples were suspended in air under slight spring tension and were tested by repeatedly passing through them a square wave 36A current pulse of 60 seconds duration separated by intervals of 45 seconds. This caused the samples to be temperature cycled up to about 750°C and back again. The samples were observed with an optical microscope during this cycling, and the formation of copper oxide was noted. Results are shown in Table 2.

10

TABLE 2

| Example | Al Intermediate layer thickness (micrometres) | Notes |
| --- | --- | --- |
| 9 (comparison) | 1 | Slight oxide formation after 1 cycle |
| | | Moderate oxide formation after 2 cycles |
| | | Severe oxide formation after 3 cycles |
| 10 | 3 | Slight oxide formation after 2 cycles |
| | | Moderate oxide formation after 4 cycles |
| 11 | 12 | Slight oxide formation after 14 cycles |
| | | Test terminated after 50 cycles; oxide formation still only slight |
| 12 | 3* | Slight oxide formation noted after 100 cycles |

\* This sample included an additional 1 micrometre layer of nickel between the copper and the aluminium.

The results demonstrate the increase in resistance to substrate oxidation as the thickness of the intermediate layer increases, and the added improvement obtained by the provision of an additional nickel layer.

Examples 13 and 14

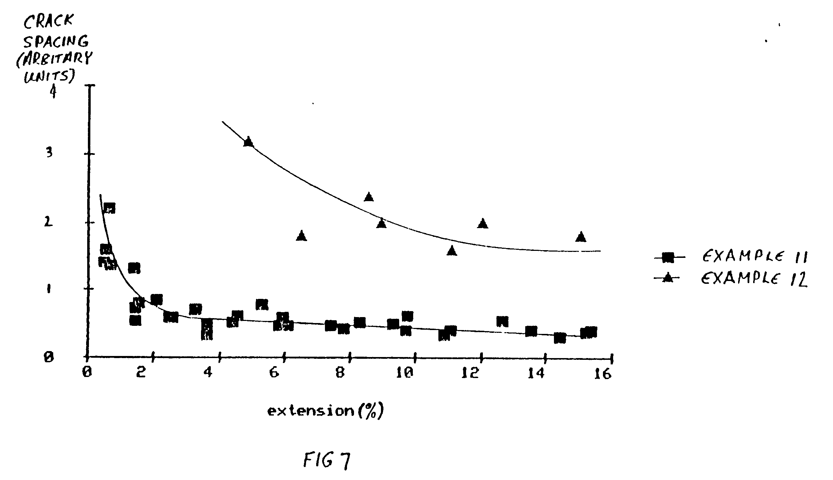

The wire samples prepared in Examples 11 and 12 were subjected to a high tensile strain and the formation of cracks in the surface aluminium oxide layer was observed, and the results are shown graphically in Figure 7. As the strain is increased the spacing between cracks, shown by the ordinate, decreases as the strain, shown by the abscissa, increases. It can be seen that, for any given strain, the number of cracks in the sample employing a 12 micrometre intermediate aluminium layer (Example 12) was considerably smaller than the number of cracks in the sample employing a 3 micrometre intermediate aluminium layer (Example 11). The reduction in crack density indicates a change in the detailed stress distribution in the refractory layer as the sample is stretched. This is thought to be an important feature in the reduction of spalling during mechanical abuse.

Examples 15 to 17

Samples of 19 strand 22 AWG copper wire conductor were coated with aluminium intermediate layers as described in Examples 1—7, and subsequently coated with refractory aluminium nitride. The nitride refractory layers were deposited by reactive sputtering from an aluminium target in an argon/nitrogen atmosphere at a total pressure of $8.10^{-3}$ mbar. The flow of nitrogen to the chamber was maintained so that stoichiometric aluminium nitride was deposited onto the conductor. The wire residence time and target power were adjusted so that the nitride refractory coating thickness was about 2 micrometres. The aluminium intermediate layers were of different thickness, as shown in Table 3.

The dielectric strength of the refractory aluminium nitride layer was measured to be 108 V/μm.

The high temperature electrical performance of the insulated wires was tested as described in Examples 1—7. The results are shown in Table 3. It can be seen that wires provided with metallic intermediate layers beneath the nitride refractory coating perform significantly better at high temperatures.

The tested samples were examined with an optical microscope. Those with a thick aluminium intermediate layer (Example 16) were seen to be in good condition, with no circumferential cracking of the nitride layer. Those with no intermediate layer (Example 15) were badly cracked, and spalled away from the copper conductor.

11

EP 0 170 440 B1

TABLE 3

| Example | Aluminium intermediate layer thickness (micrometres) | Time to failure 900°C propane flame (min.) |
|---|---|---|
| 15 | 0 | 0.2 |
| 16 | 10 | 90 |
| 17 | 3* | 39 |

* This sample was provided with a layer of graded stoichiometry between the aluminium intermediate layer and the nitride refractory coating. The stoichometry varied smoothly from aluminium metal to aluminium nitride, and the total thickness of the graded layer plus the stoichiometric nitride layer was about 2 micrometres.

Examples 18 and 19

Refractory aluminium oxide coatings 3.5 micrometres thick were sputtered onto 19 strand 22 AWG copper wire conductors, some of which had been provided with an aluminium intermediate layer 3 micrometres thick according to the invention. The adhesion of the refractory layer to the underlying metal was assessed by observing the samples with an optical microscope whilst stretching them. At a particular strain, the adhesion of the refractory layer is overcome, and it spalls away from the underlying metal. The strain at which this occurred was noted; the results are given in Table 4. It can be seen that it is possible to choose intermediate layers that significantly enhance the adhesion of the refractory layer to the underlying metal. It was observed that the refractory layer of Example 18 would spall away with gentle handling whereas the layer of Example 19 could withstand severe mechanical abuse without spalling.

TABLE 4

| Example | Aluminium intermediate layer thickness (micrometres) | Strain at spalling (%) |
|---|---|---|
| 18 (comparison) | 0 | 1.6 |
| 19 | 3 | 6.7 |

Examples 20 and 21

Samples of 19 strand 22 AWG copper wire conductors were sputter coated to a thickness of 5 micrometres with a refractory layer of silicon dioxide by RF sputtering from a silicon dioxide target. In some cases this refractory layer was applied directly to the copper conductor, in others the wires were provided with an aluminium intermediate layer 10 micrometres thick, manufactured as described in Examples 1 to 7. The high temperature electrical performance of twisted pairs of these wires was tested as described above. The results are shown in Table 5, from which it can be seen that wires provided with a metallic intermediate layer perform significantly better than those without the intermediate layer. After testing, the wires were again examined with a microscope. It was found that the refractory layer of Example 20 (no intermediate layer) was badly cracked and spalled; that of Example 21 (with aluminium intermediate layer) was neither cracked nor spalled. Failure seemed to have occurred by slow growth of copper oxide between the strands.

TABLE 5

| Example | Aluminium intermediate layer thickness (μm) | Time to failure in a 900°C propane flame (min.) |
|---|---|---|
| 20 (comparison) | 0 | 1 |
| 21 | 10 | 142 |

12

# EP 0 170 440 B1

Example 22

Example 7 was repeated with the exception that the aluminium intermediate layer had a thickness of 10 micrometres and that the wire was provided with a cross-linked polyethylene/ethylene vinyl acetate copolymer blend insulation of 0.25 mm thickness. The wire survived 113 minutes before failure in a propane flame at a nominal temperature of 900°C. The temperature is given as a nominal temperature only in view of the uncontrolled temperature rise experienced by the wire as the polymeric insulation ignited.

Examples 23 and 24

Samples of flat copper conductor were sputter coated with 2 µm of chromium, followed by 2 µm of aluminium oxide. As a comparison, bare flat copper conductors (with no chromium intermediate layer) were also sputter coated with 2 µm of aluminium oxide. The insulated conductors were tested for their high temperature integrity by heating them (in air) to 800°C for ten minutes. The samples were then examined using an optical microscope.

It was found that the samples provided with the chromium intermediate layer (Example 23) had survived perfectly intact: neither spalling nor copper oxide growth was visible. In contrast, those provided with no intermediate layer (Example 24) were in very poor condition. The aluminium oxide film had completely disappeared, and the copper conductor was covered with a thick scale of copper oxide.

Examples 25 and 26

19 Strand, 22 AWG copper wire conductors were provided with sputtered nickel intermediate layers, extending around the bundle but not around the individual strands, of thicknesses listed in Table 6. A sputtered refractory layer of aluminium oxide about 4 µm thick was then added, using the method described in Examples 1—7.

The high temperature electrical performance of the insulated wires was tested as described in Examples 1—7: the results were given in Table 6, from which it can be seen that nickel intermediate layers (particularly thicker ones) according to the invention considerably improve the wires' high temperature performance.

TABLE 6

| Example | Nickel intermediate layer thickness (µm) | Time to failure in a 900°C propane flame (min.) |
|---|---|---|
| 1 (comparison) | 0 | 0.2 |
| 25 | 1.5 | 6 |
| 26 | 3 | 17 |

## Claims

1. An electrical wire which has a conductor (1, 21) formed from metallic copper, the conductor having on its surface an adherent substantially contaminant-free electrically insulating refractory layer (4, 25) and an intermediate layer (2) formed from a metal that acts as a barrier to diffusion of oxygen or copper or both, the intermediate layer having a thickness of at least 1.5 micrometres so that migration of copper through the refractory layer when the article is heated is suppressed, the wire also having an outer polymeric insulation (3, 6).

2. An electrical wire which has a conductor (1, 21) formed from metallic copper, the conductor having on its surface an adherent substantially contaminant-free refractory layer (4, 25) and an adherent intermediate layer (2) formed from a metal that has a lower modulus than that of copper, the intermediate layer having a thickness of at least 1.5 micrometres, the wire also having an outer polymeric insulation (3, 6).

3. A wire as claimed in claim 1 or claim 2 wherein the thickness of the intermediate layer (2) is at least 2.0 micrometres.

4. A wire as claimed in any one of claims 1 to 3, wherein the refractory layer (4, 25) comprises a metal oxide or a metal nitride.

5. A wire as claimed in any one of claims 1 to 4, wherein the refractory layer (4, 25) has been formed by a vacuum deposition process and preferably a sputter plating method.

6. A wire as claimed in any one of claims 1 to 5, wherein the intermediate layer (2) has been formed by a vacuum deposition technique and preferably by a sputter plating method, by a metal rolling or electroplating method or by coating from a metal melt.

7. A wire as claimed in any one of claims 1 to 6, wherein the intermediate layer (2) is formed from

13

aluminium, silicon, titanium, tantalum, nickel, manganese, chromium or an alloy thereof.

8. A wire as claimed in any one of claims 1 to 7, wherein the refractory layer (4, 25) comprises an inorganic metal compound and the intermediate layer (2) comprises the same metal as that present in the refractory layer.

9. A wire as claimed in any one of claims 1 to 8, wherein the intermediate layer (2) is formed from a metal that forms an intermetallic compound or solid solution with copper when heated.

10. A wire as claimed in any one of claims 1 to 9, wherein the intermediate layer (2) comprises aluminium or nickel.

11. A wire as claimed in any one of claims 1 to 12, wherein the refractory layer (4, 25) comprises an oxide of aluminium, silicon, titanium, or tantalum or a nitride of silicon or aluminium.

12. A wire as claimed in any one of claims 1 to 11, wherein the refractory layer (4, 25) has a thickness of at least 1, preferably at least 2, more preferably at least 5 and especially at least 10 micrometres.

13. A wire as claimed in any one of claims 1 to 12, wherein the refractory layer (3, 24) has a stoichiometry that varies throughout at least part of its thickness such that the proportion of metal or semi-metal in the layer decreases towards the outer surface of the layer.

14. A wire as claimed in claim 13, wherein the stoichiometry of the refractory layer varies such that there is no defined boundary between the intermediate layer and the refractory layer.

15. A wire as claimed in any one of claims 1 to 14, which has one or more additional layers (5, 26) on top of the refractory layer.

16. A wire as claimed in any one of claims 1 to 15, which contains more than one intermediate layer (23, 24).

17. A wire as claimed in claim 16, wherein at least one of the intermediate layers (22, 23) acts as a barrier to diffusion of copper and/or oxygen, and the or at least one other intermediate layer acts as a strain relieving layer and/or a keying layer.

## Patentansprüche

1. Elektrischer Draht, der einen Leiter (1, 21) aus metallischem Kupfer aufweist, wobei der Leiter an seiner Oberfläche eine anhaftende, im wesentlichen von Verunreinigungen freie, elektrisch isolierende feuerfeste Schicht (4, 25) und eine Zwischenschicht (2) aufweist, die aus einem Metall gebildet ist, welches als Barriere gegen die Diffusion von Sauerstoff oder Kupfer oder beiden wirkt, wobei die Zwischenschicht eine Dicke von mindestens 1,5 Mikrometer hat, so daß eine Wanderung von Kupfer durch die feuerfeste Schicht unterdrückt wird, wenn der Gegenstand erhitzt wird, wobei der Draht außerdem eine äußere polymere Isolierung (3, 6) aufweist.

2. Elektrischer Draht, der einen Leiter (1, 21) aus metallischem Kupfer aufweiste, wobei der Leiter an seiner Oberfläche eine anhaftende, im wesentlichen von Verunreinigungen freie feuerfeste Schicht (4, 25) und eine anhaftende Zwischenschicht (2) aufweist, die aus einem Metall gebildet ist, das einen niedrigeren Modul als Kupfer hat, wobei die Zwischenschicht eine Dicke von mindestens 1,5 Mikrometer besitzt, wobei der Draht außerdem eine äußere polymere Isolierung (3, 6) hat.

3. Draht nach Anspruch 1 oder Anspruch 2, wobei die Dicke der Zwischenschicht (2) mindestens 2,0 Mikrometer beträgt.

4. Draht nach einem der Ansprüche 1 bis 3, wobei die feuerfeste Schicht (4, 25) ein Metalloxid oder ein Metallnitrid aufweist.

5. Draht nach einem der Ansprüche 1 bis 4, wobei die feuerfeste Schicht (4, 25) durch ein Vakuumabscheidungsverfahren und vorzugsweise ein Zerstäubungsbeschichtungsverfahren gebildet worden ist.

6. Draht nach einem der Ansprüche 1 bis 5, wobei die Zwischenschicht (2) durch eine Vakuumabscheidungstechnik und vorzugsweise durch ein Zerstäubungsbeschichtungsverfahren, durch ein Metallwalzverfahren oder durch ein Galvanisierungsverfahren oder aber durch eine Beschichtung aus einer Metallschmelze gebildet worden ist.

7. Draht nach einem der Ansprüche 1 bis 6, wobei die Zwischenschicht (2) aus Aluminium, Silizium, Titan, Tantal, Nickel, Mangan, Chrom oder aus einer Legierung von diesen gebildet ist.

8. Draht nach einem der Ansprüche 1 bis 7, wobei die feuerfeste Schicht (4, 25) eine anorganische Metallverbindung aufweist und die Zwischenschicht (2) das gleiche Metall enthält, das in der feuerfesten Schicht vorhanden ist.

9. Draht nach einem der Ansprüche 1 bis 8, wobei die Zwischenschicht (2) aus einem Metall gebildet ist, welches bei Erhitzung eine intermetallische Verbindung oder eine feste Lösung mit Kupfer bildet.

10. Draht nach einem der Ansprüche 1 bis 9, wobei die Zwischenschicht (2) Aluminium oder Nickel aufweist.

11. Draht nach einem der Ansprüche 1 bis 10, wobei die feuerfeste Schicht (4, 25) ein Oxid von Aluminium, Silizium, Titan oder Tantal oder ein Nitrid von Silizium oder Aluminium aufweist.

12. Draht nach einem der Ansprüche 1 bis 11, wobei die feuerfeste Schicht (4, 25) eine Dicke von mindestens 1 µm, vorzugsweise von mindestens 2 µm, noch bevorzugter von mindestens 5 µm und insbesondere von mindestens 10 µm aufweist.

14

13. Draht nach einem der Ansprüche 1 bis 12, wobei die feuerfeste Schicht (3, 24) eine Stöchiometrie aufweist, die sich zumindest über einen Teil ihrer Dicke ändert, so daß der Anteil von Metall oder Halbmetall in der Schicht zur Außenoberfläche der Schicht hin abnimmt.

14. Draht nach Anspruch 13, wobei die Stöchiometrie der feuerfesten Schicht sich so ändert, daß es keine definierte Grenzschicht zwischen der Zwischenschicht und der feuerfesten Schicht gibt.

15. Draht nach einem der Ansprüche 1 bis 14, der eine oder mehrere zusätzliche Schichten (5, 26) auf der Oberseite der feuerfesten Schicht aufweist.

16. Draht nach einem der Ansprüche 1 bis 15, der mehr als eine Zwischenschicht (23, 24) enthält.

17. Draht nach Anspruch 16, wobei zumindest eine der Zwischenschichten (22, 23) als Barriere gegen die Diffusion von Kupfer und/oder Sauerstoff wirkt und wobei die oder mindestens eine andere Zwischenschicht als Spannungsentlastungsschicht und/oder als Verkeilungsschicht wirkt.

**Revendications**

1. Fil électrique qui comprend un conducteur (1, 21) formé de cuivre métallique, le conducteur portant sur sa surface une couche réfractaire adhérente (4, 25) électriquement isolante et pratiquement dépourvue d'impuretés et une couche intermédiaire (2) formée d'un métal qui joue le rôle de barrière à la diffusion de l'oxygène ou du cuivre, ou bien de ces deux éléments, la couche intermédiaire ayant une épaisseur d'au moins 1,5 micromètre, de sorte que la migration du cuivre à travers la couche réfractaire lorsque l'article est chauffé est supprimée, le fil comprenant également un isolant polymérique extérieur (3, 6).

2. Fil électrique qui comprend un conducteur (1, 21) formé de cuivre métallique, le conducteur portant sur sa surface une couche réfractaire adhérente (4, 25) pratiquement dépourvue d'impuretés et une couche intermédiaire adhérente (2) formée d'un métal qui possède un module inférieur à celui du cuivre, la couche intermédiaire ayant une épaisseur d'au moins 1,5 micromètre, le fil comprenant également un isolant polymérique extérieur (3, 6).

3. Fil suivant la revendication 1 ou la revendication 2, dans lequel l'épaisseur de la couche intermédiaire (2) est au moins égale à 2,0 micromètres.

4. Fil suivant l'une quelconque des revendications 1 à 3, dans lequel la couche réfractaire (4, 25) consiste en un oxyde métallique ou un nitrure métallique.

5. Fil suivant l'une quelconque des revendications 1 à 4, dans lequel la couche réfractaire (4, 25) a été formée par un procédé de dépôt sous vide et, de préférence, un procédé de placage par pulvérisation cathodique.

6. Fil suivant l'une quelconque des revendications 1 à 5, dans lequel la couche intermédiaire (2) a été formée par une technique de dépôt sous vide et, de préférence, par un procédé de placage par pulvérisation cathodique, par un procédé de laminage ou d'électrodéposition d'un métal ou bien par revêtement à partir d'une masse métallique fondue.

7. Fil suivant l'une quelconque des revendications 1 à 6, dans lequel la couche intermédiaire (2) est formée d'aluminium, de silicium, de titane, de tantale, de nickel, de manganèse, de chrome ou d'un de leurs alliages.

8. Fil suivant l'une quelconque des revendications 1 à 7, dans lequel la couche réfractaire (4, 25) comprend un composé métallique inorganique et la couche intermédiaire (2) comprend un métal identique à celui qui est présent dans la couche réfractaire.

9. Fil suivant l'une quelconque des revendications 1 à 8, dans lequel la couche intermédiaire (2) est constituée d'un métal qui forme un composé intermétallique ou une solution solide avec le cuivre lorsqu'il est chauffé.

10. Fil suivant l'une quelconque des revendications 1 à 9, dans lequel la couche intermédiaire (2) consiste en aluminium ou en nickel.

11. Fil suivant l'une quelconque des revendications 1 à 12, dans lequel la couche réfractaire (4, 25) consiste en un oxyde d'aluminium, de silicium, de titane ou de tantale ou bien en un nitrure de silicium ou d'aluminium.

12. Fil suivant l'une quelconque des revendications 1 à 11, dans lequel la couche réfractaire (4, 25) possède une épaisseur d'au moins 1, avantageusement d'au moins 2, de préférence d'au moins 5 et notamment d'au moins 10 micromètres.

13. Fil suivant l'une quelconque des revendications 1 à 12, dans lequel la couche réfractaire (3, 24) possède une stoechiométrie qui varie dans au moins une partie de son épaisseur, de sorte que la proportion de métal ou semi-métal dans la couche diminue vers la surface extérieure de la couche.

14. Fil suivant la revendication 13, dans lequel la stoechiométrie de la couche réfractaire varie de sorte qu'il n'existe aucune limite définie entre la couche intermédiaire et la couche réfractaire.

15. Fil suivant l'une quelconque des revendications 1 à 14, qui comprend une ou plusieurs couches supplémentaires (5, 26) au-dessus de la couche réfractaire.

16. Fil suivant l'une quelconque des revendications 1 à 15, qui contient plus d'une couche intermédiaire (23, 24).

17. Fil suivant la revendication 16, dans lequel au moins l'une des couches intermédiaires (22, 23) joue le rôle de barrière à la diffusion du cuivre et/ou de l'oxygène et la, ou au moins une autre, couche intermédiaire joue le rôle de couche de relaxation de contrainte et/ou de couche de liaison.

Fig. 1.

Fig. 2.

Fig. 3.

Fig. 4.

VACUUM
PUMP

2

FIG 5.

FIG 6

FIG 7